# EUROPEAN PATENT APPLICATION

(11) **EP 4 656 647 A1**
(43) Date of publication of application: **03.12.2025**
(21) Application number: 25179022.6
(22) Date of filing: 27.05.2025
(51) Int. Cl.: C07F 15/00, A61B 5/1486

(54) **TRANSITION METAL COMPLEX AND METHODS FOR ITS PREPARATION AND USE**

(30) Priority: 29.05.2024 CN 202410688634
(71) Applicant: Shanghai United Imaging Microelectronics Technology Co., Ltd., Shanghai 201800 (CN)
(72) Inventor: SUN, Yue, Shanghai, 201800 (CN)
(74) Representative: Murgitroyd & Company

(57) **Abstract**

A transition metal complex is provided, which has a formula selected from: [M(A)(B)(C)]^{a}bY,[M(A)(D)(E)(F)]^{a}bY, or [M(A)(G)(H)(O)(P)]^{a}bY; wherein:M is a transition metal element; A is a bidentate ligand containing a benzene ring and a carbene heterocyclic ring with at least one nitrogen atom; B, C, and D are each independently a bidentate ligand selected from the group consisting of a structure represented by Formula (1) and a structure represented by Formula (2), as shown below; E, F, G, H, O, and P are each independently a monodentate ligand selected from a heterocyclic ring containing at least one heteroatom, CN⁻, or Cl⁻; 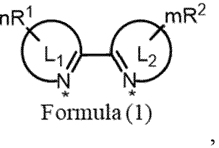 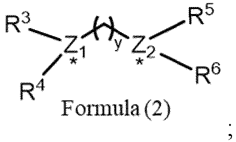 in Formula (1), R¹ and R² are each independently selected from a hydrogen atom, an alkyl group, an alkoxy group, or an alkylamino group;in Formula (2), R³, R⁴, R⁵, and R⁶are each independently selected from an alkyl group, a substituted or an unsubstituted aryl group.

## Description

### CROSS-REFERENCE TO RELATED APPLICATION

This application claims priority to Chinese Patent Application No. 202410688634.4, filed on May 29, 2024.

### TECHNICAL FIELD

The present application relates to sensors, and in particular, to transition metal complexes, methods for their preparation, and uses thereof.

### BACKGROUND

Continuous glucose monitoring (CGM) is a technology that monitors fluctuations in a subject's glucose concentration using a glucose sensor, such as through subcutaneous implantation of microelectrodes. Current CGM systems typically employ glucose sensors based on enzyme electrode technology to measure blood glucose levels in the human body. In existing glucose sensors, common electron transfer media include compounds such as potassium ferricyanide, ruthenium amines, and metal complexes containing N-N ligands. However, compounds like potassium ferricyanide and ruthenium amines suffer from limitations such as poor stability and mismatched reactivity. Metal complexes with N-N ligands often using nitrogen-based ligands like bipyridine or o-phenanthroline require higher operating voltages due to their inability to effectively lower the redox potential, making them susceptible to interference from numerous substances.

Metal complexes incorporating C-N ligands can reduce the redox potential while maintaining reactivity, which is particularly advantageous for metals commonly used in glucose sensors, such as osmium, by increasing the number of C-N ligand interactions. However, current methods for adjusting the number of C-N ligands in synthesis rely on toxic mercury-containing compounds, presenting significant environmental and safety concerns.

Therefore, it is desirable to provide an transition metal complex methods for its preparation and use.

### SUMMARY

An aspect of the present application may provide a transition metal complex, wherein the transition metal complex formula is selected from: [M(A)(B)(C)]^{a}bY, [M(A)(D)(E)(F)]^{a}bY, or [M(A)(G)(H)(O)(P)]^{a}bY; wherein: M is a transition metal element; A is a bidentate ligand containing a benzene ring and a carbene heterocyclic ring with at least one nitrogen atom; B, C, and D are each independently a bidentate ligand selected from the group consisting of a structure represented by Formula (1) and a structure represented by Formula (2), as shown below; E, F, G, H, O, and P are each independently a monodentate ligand selected from a heterocyclic ring containing at least one heteroatom, CN⁻, or Cl⁻; a represents the number of positive charges and a is 0 or 1; Y is a counterion; b represents the number of counterions and b is 0 or 1; 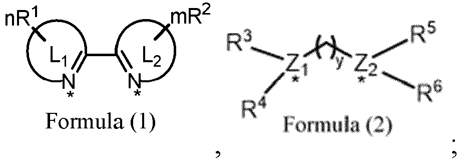 in Formula (1), L₁ and L₂ are each independently selected from heterocycles containing at least one nitrogen atom, R¹ and R² are each independently selected from a hydrogen atom, an alkyl group, an alkoxy group, or an alkylamino group, and n and m are each independently selected from integers from 0 to 5; in in Formula (2), Z₁ and Z₂ are each independently selected from N, P, or As; R³, R⁴, R⁵, and R⁶ are each independently selected from an alkyl group, a substituted or an unsubstituted aryl group, and y is an integer from 1 to 4.

In some embodiments, A is a bidentate ligand having the structure shown in Formula (3), 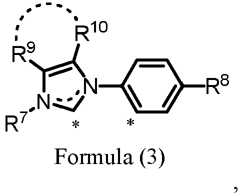 in Formula (3) R⁷ is selected from an alkyl group, a substituted or an unsubstituted benzyl group ; R⁸ is selected from a hydrogen atom, an alkyl group, or an alkoxy group, and there may or may not be a covalent bond between R⁹ and R¹⁰ ; R⁹ and R¹⁰ are each independently selected from a hydrogen atom, an alkyl group, or an aryl group.

In some embodiments, R⁷ is selected from an alkyl group with 1 to 4 carbon atoms or a benzyl group with at least one hydrogen atom replaced by an electron group, wherein the electron group is selected from an alkyl group with 1 to 4 carbon atoms, an alkoxy group with 1 to 3 carbon atoms, a cyano group, a carboxyl group, an aldehyde group, or a halogen atom.

In some embodiments, R⁸ is selected from an alkyl group with 1 to 2 carbon atoms or an alkoxy group with 1 to 2 carbon atoms.

In some embodiments, R⁹ and R¹⁰ are each independently selected from alkyl groups with 1 to 2 carbon atoms.

In some embodiments, R¹ and R² are each independently selected from an alkyl group with 1 to 3 carbon atoms, an alkoxy group with 1 to 3 carbon atoms, and an alkylamino group with 1 to 6 carbon atoms.

In some embodiments, R³, R⁴, R⁵, and R⁶ are each independently selected from an alkyl group with 3 to 6 carbon atoms or an aryl group with at least one hydrogen atom replaced by an electron-donating group with 1 to 4 carbon atoms, and the electron-donating group is selected from an alkyl group, an alkoxy group, or an alkylamino group.

In some embodiments, the heterocyclic ring containing at least one heteroatom is selected from at least one of pyridine, imidazole, pyrazole, oxazole, thiazole, pyrazine, triazole, pyrimidine.

In some embodiments, the transition metal element is selected from Fe, Ru, Os, Co, Rh, or Ir.

In some embodiments, the counterion is selected from at least one of PF₆⁻, BF₄⁻, Cl⁻, I⁻, F⁻, or Br⁻.

In some embodiments, the transition metal complex is any of the compounds having the structure shown in Formulas (4) to (10):

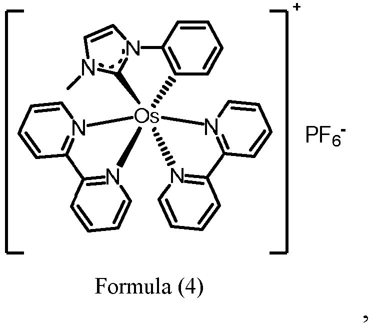

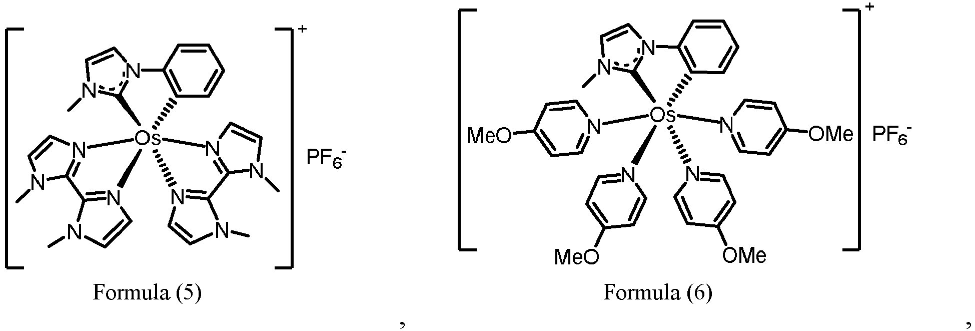

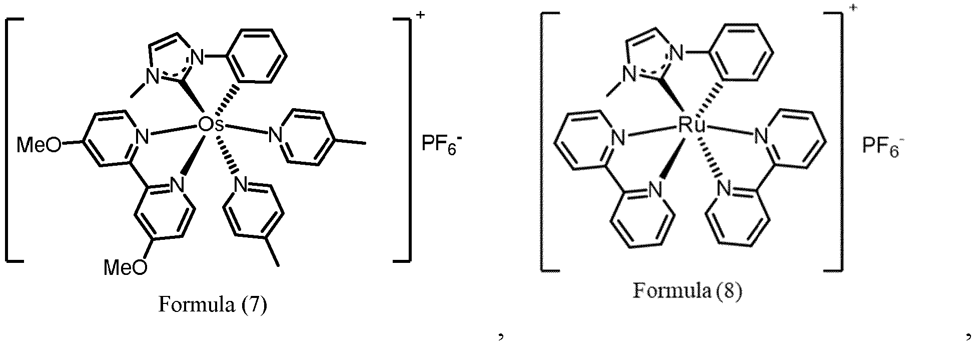

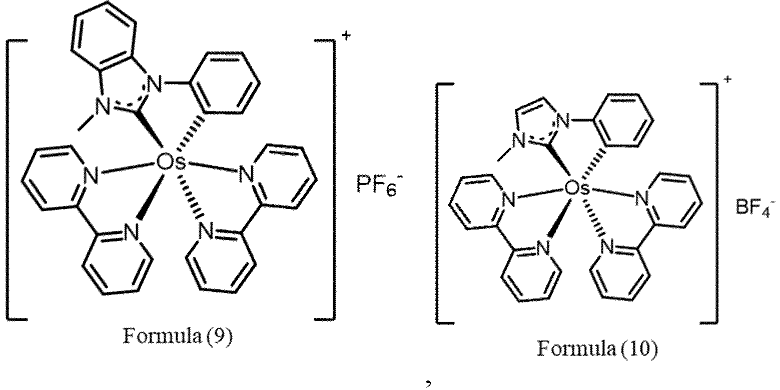

Another aspect of the present application may provide a method for preparing the transition metal complex, which comprises: under a protective atmosphere, mixing and reacting the precursor material of ligand A, an additive, a transition metal dimer, a basic compound, and a first organic solvent, and separating to obtain the precursor of the transition metal complex containing ligand A; under a protective atmosphere, mixing and reacting the precursor of the transition metal complex containing ligand A, a precursor material of ligand S, and a second organic solvent, then adding the counterion precursor material for reaction, and after separation and drying, obtaining the transition metal complex; wherein, the ligand S comprises a ligand B and a ligand C, or the ligand S comprises a ligand D, a ligand E, and a ligand F, or the ligand S comprises a ligand G, a ligand H, a ligand O, and a ligand P.

In some embodiments, at least one of the following conditions must be met in the steps for preparing the transition metal complex:
(1) the molar ratio of the precursor material of ligand A to the additive is 2:1 to 4:1, the molar ratio of the precursor material of ligand A to the transition metal dimer is 2:1 to 4:1, and the molar ratio of the precursor material of ligand A to the basic compound is 1:2 to 1:80;
(2) the additive is selected from at least one of silver oxide, silver trifluoromethanesulfonate, silver carbonate, or molecular sieve, the transition metal dimer is selected from aryl osmium dimer, and the basic compound is selected from at least one of basic aluminum oxide, sodium carbonate, or sodium bicarbonate;
(3) in the steps of mixing and reacting the precursor material of ligand A, the additive, the transition metal dimer, the basic compound, and the first organic solvent, the reaction temperature is 25 °C to 40 °C and the reaction time is 12h to 36h;
(4) the molar ratio of the transition metal complex precursor containing ligand A to the precursor material of ligand S is 1: 1 to 1:4;
(5) the molar ratio of the transition metal complex precursor containing ligand A to the counterion precursor material is 1: 10 to 1:30;
(6) in the steps of mixing and reacting the transition metal complex precursor containing ligand A, a precursor material of ligand S, and the second organic solvent, the second organic solvent is selected from at least one of ethylene glycol, methanol, and ethanol, the reaction temperature is 25 °C to 40 °C, and the reaction time is 12h to 36h.

Another aspect of the present application may provide an electrochemical biosensor device, wherein the electrochemical biosensor device comprises a sensing membrane, the sensing membrane comprising a bioanalytical enzyme and an electron transfer medium, wherein the electron transfer medium is the transition metal complex as described above.

In some embodiments, the bioanalytical enzyme is selected from an oxidase or a dehydrogenase.

Additional features will be set forth in part in the description which follows, and in part will become apparent to those skilled in the art upon examination of the following and the accompanying drawings or may be learned by production or operation of the examples. The features of the present application may be realized and attained by practice or use of various aspects of the methodologies, instrumentalities, and combinations set forth in the detailed examples discussed below.

### BRIEF DESCRIPTION OF THE DRAWINGS

The present application is further illustrated in terms of exemplary embodiments. These exemplary embodiments are described in detail with reference to according to the drawings. These embodiments are non-limiting exemplary embodiments, in which like reference numerals represent similar structures, and wherein:
FIG. 1 is cyclic voltammetry curve showing the electrochemical properties of the osmium complex prepared in Example 1 of the present application.
FIG. 2 is cyclic voltammetry curve showing the electrochemical properties of the osmium complex prepared in Example 2 of the present application.
FIG. 3 is cyclic voltammetry curve showing the electrochemical properties of the osmium complex prepared in Example 8 (Proportional Example 1) of the present application.
FIG. 4 is graphs depicting the relationship between electrochemical catalytic oxidation current and glucose concentration, as well as current density and time, for a sensing membrane using the osmium complex of Example 1 as the electron transfer medium.
FIG. 5 is graphs depicting the relationship between electrochemical catalytic oxidation current and glucose concentration, as well as current density and glucose concentration, for a sensing membrane using the osmium complex of Embodiment 1 as the electron transfer medium.
FIG. 6 is graphs depicting the relationship between electrochemical catalytic oxidation current and glucose concentration, as well as current density and time, for a sensing membrane using the osmium complex of Example 2 as the electron transfer medium.
FIG. 7 is graphs depicting the relationship between electrochemical catalytic oxidation current and glucose concentration, as well as current density and glucose concentration, for a sensing membrane using the osmium complex of Example 2 as the electron transfer medium.
FIG. 8 is graphs depicting the relationship between the electrochemical catalytic oxidation current density and time on a sensing membrane prepared using the osmium complex of Example 8 (Proportional Example 1) of the present invention as an electron transfer medium, with varying glucose concentrations.
FIG. 9 is graphs depicting the relationship between electrochemical catalytic oxidation current and glucose concentration, as well as current density and glucose concentration, for a sensing membrane using the osmium complex of Example 8 (Proportional Example 1) as the electron transfer medium of the present application.

### DETAILED DESCRIPTION

In the following detailed description, numerous specific details are set forth by way of examples in order to provide a thorough understanding of the relevant disclosure. However, it should be apparent to those skilled in the art that the present application may be practiced without such details. In other instances, well-known methods, procedures, systems, components, and/or circuitry have been described at a relatively high level, without detail, in order to avoid unnecessarily obscuring aspects of the present application. Various modifications to the disclosed embodiments will be readily apparent to those skilled in the art, and the general principles defined herein may be applied to other embodiments and applications without departing from the spirit and scope of the present application. Thus, the present application is not limited to the embodiments shown, but is to be accorded the widest scope consistent with the claims.

The terminology used herein is for the purpose of describing particular example embodiments only and is not intended to be limiting. As used herein, the singular forms "a", "an", and "the" may be intended to include the plural forms as well, unless the context clearly indicates otherwise. It will be further understood that the terms "comprise", "comprises", and/or "comprising", "include", "includes", and/or "including", when used in this specification, specify the presence of stated features, integers, steps, operations, elements, and/or components, but do not preclude the presence or addition of one or more other features, integers, steps, operations, elements, components, and/or groups thereof.

It will be understood that the terms "system", "engine", "unit", "module", and/or "block" used herein are one method to distinguish different components, elements, parts, sections or assemblies of different levels in ascending order. However, the terms may be displaced by another expression if they achieve the same purpose.

It will be understood that when a unit, engine, module, or block is referred to as being "on", "connected to", or "coupled to", another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items. The terms "pixel" and "voxel" in the present application are used interchangeably to refer to an element of an image.

These and other features, and characteristics of the present application, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present application. It is understood that the drawings are not to scale.

To facilitate an understanding of the present application, the following provides a detailed description. It should be appreciated, however, that the present application may be embodied in many different forms and is not limited to the embodiments or examples set forth herein. Rather, these embodiments are provided to ensure a thorough and complete disclosure of the application as described.

Unless otherwise defined, all technical and scientific terms used herein have the same meaning as commonly understood by one of ordinary skill in the art to which this application belongs. The terminology used in the description of the application herein is for the purpose of describing specific embodiments only and is not intended to limit the application. As used herein, the term "and/or" includes any and all combinations of one or more of the associated listed items.

Continuous glucose monitoring (CGM) is a technology that tracks glucose concentration changes in a subject's body using subcutaneous microelectrodes and other means via a glucose sensor. Current CGM systems typically employ glucose sensors based on enzyme electrode technology to measure blood glucose levels. In existing glucose sensors, common electron transfer media include compounds such as potassium ferricyanide, ruthenium amines, and metal complexes with N-N ligands. However, compounds like potassium ferricyanide and ruthenium amines suffer from limitations such as poor stability and mismatched reactivity. Metal complexes with N-N ligands often utilizing ligands like bipyridine or o-phenanthroline require higher operating voltages because these ligands are ineffective at lowering the redox potential, making the sensors susceptible to interference from numerous substances.

While metal complexes incorporating C-N ligands have emerged, which can reduce the redox potential while maintaining reactivity, the underlying mechanism is as follows: N-N ligands (e.g., bipyridine) act as π-acid ligands, both donating electrons to the central metal and accepting back-donated π-electrons. In contrast, C-N ligands form carbon-metal bonds with the central metal, serving as σ-donors with stronger electron-donating capabilities. Importantly, increasing the number of C-N ligands, i.e., increasing carbon-metal bond formation further lowers the redox potential. Thus, multiple carbon-metal bonds are highly advantageous for redox potential tuning. However, for metals commonly used in glucose sensors, such as osmium, increasing C-N ligands to enhance carbon-metal bonds has historically required toxic mercury-containing compounds in synthesis, posing significant environmental risks.

In this application, a bidentate ligand containing a benzene ring and a carbene heterocycle with at least one nitrogen atom coordinates with the transition metal, enabling the simultaneous formation of two carbon-metal bonds. This introduces C-C-type ligands (carbon-metal bonded ligands) into the transition metal complex. Compared to C-N-type ligands, C-C-type ligands exhibit stronger electron-donating ability, which is critical for reducing the redox potential of the complex. This facilitates easy tuning of the redox potential between -200 mV and 100 mV, enhancing electron transfer capability. Additionally, the C-C-type ligand can coordinate with other ligands to form a six-coordinate transition metal complex, providing structural stability that further improves electron transport. Importantly, the synthesis of the transition metal complex eliminates the need for toxic mercury-containing reagents, offering a simple, environmentally friendly, and cost-effective process.

When used as an electron transfer medium, the transition metal complex of the present application features a low redox potential and robust electron transfer ability. Its synthesis is free of toxic mercury reagents, simple, eco-friendly, and economical.

An aspect of the present application may provide a transition metal complex, wherein the transition metal complex formula is selected from: [M(A)(B)(C)]^{a}bY, [M(A)(D)(E)(F)]^{a}bY, or [M(A)(G)(H)(O)(P)]^{a}bY; wherein: M is a transition metal element; A is a bidentate ligand containing a benzene ring and a carbene heterocyclic ring with at least one nitrogen atom; B, C, and D are each independently a bidentate ligand selected from the group consisting of a structure represented by Formula (1) and a structure represented by Formula (2), as shown below; E, F, G, H, O, and P are each independently a monodentate ligand selected from a heterocyclic ring containing at least one heteroatom, CN⁻, or Cl⁻; a represents the number of positive charges and a is 0 or 1; Y is a counterion; b represents the number of counterions and b is 0 or 1; 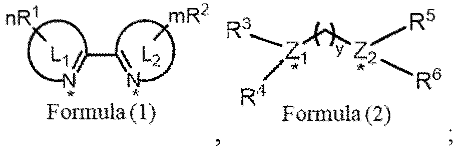 in Formula (1), L₁ and L₂ are each independently selected from heterocycles containing at least one nitrogen atom, R¹ and R² are each independently selected from a hydrogen atom, an alkyl group, an alkoxy group, or an alkylamino group, and n and m are each independently selected from integers from 0 to 5; in Formula (2), Z₁ and Z₂ are each independently selected from N, P, or As; R³, R⁴, R⁵, and R⁶ are each independently selected from an alkyl group, a substituted or an unsubstituted aryl group, and y is an integer from 1 to 4.

The transition metal complex takes the transition metal element M as the central metal atom. The complex employs a bidentate ligand containing a benzene ring and at least one nitrogen atom in a carbene heterocycle. The bidentate ligands coordinate with the transition metal element M. The coordination process can simultaneously form two carbon-metal bonds. This enables the transition metal complex to have C-C type ligands. This C-C ligand has a stronger electron-donating ability compared to the C-N ligand. The stronger electron-donating ability helps to lower the redox potential of the transition metal complex. The redox potential of transition metal complexes is thus easier to regulate. The redox potential can be regulated between -200 mV and 100 mV. This regulation enhances the electron transport capacity of the transition metal complex. The transition metal complex is thus better able to serve as an electron transport medium. Electron transfer media can be used in electrochemical sensors, especially glucose sensors.

According to the molecular formula of the transition metal complex, the present application selects a specific structure of bidentate ligand and/or monodentate ligand. The selected ligand(s) cooperate with the A ligand. The ligands and the A ligand together form a structurally stable six-coordination transition metal complex. A stable structure can further enhance the electron transport capacity of the transition metal complex. Specifically, when the molecular formula of the transition metal complex is M(A)(D)(E)(F) ^{a}bY, the structure of the transition metal complex is as shown in formula (11), 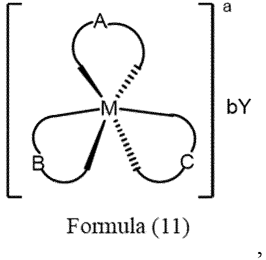 where A, B, and C are all bidentate ligands.

When the molecular formula of the transition metal complex is M(A)(D)(E)(F) ^{a}bY, the structure of the transition metal complex is as shown in Formula (12) or Formula (13), 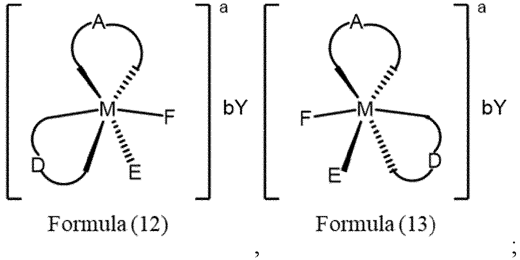 where A and D are both bidentate ligands and E and F are both monodentate ligands.

When the molecular formula of the transition metal complex is M(A)(G)(H)(O)(P) ^{a}bY, the structure of the transition metal complex is as shown in Equation (14), 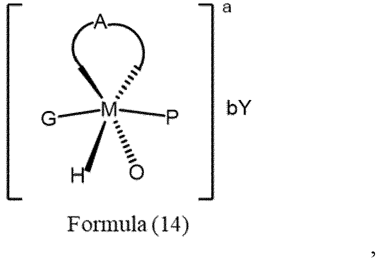 in which A is a bidentate ligand and G, H, O, and P are all monodentate ligands.

In addition, compared with metal complexes containing C - N type ligands, the transition metal complexes of the present application have significant advantages. To obtain two carbon - metal bonds in a metal complex containing C - N ligands, two C - N ligands would be synthesized. For the synthesis of two C - N ligands, for certain metal elements such as osmium, mercury - containing toxic compounds are often required. In the transition metal complex of the present application, only one ligand needs to be changed to form two carbon - metal bonds. This approach is more efficient. The synthesis process of the present application does not require mercury - containing toxic reagents. The synthesis method of the present application is simple, and the synthesis process of the present application is environmentally friendly and economical.

Therefore, when the transition metal complex of the present application is used as an electron transfer medium, it has the characteristics of low redox potential and strong electron transfer ability. At the same time, no mercury - containing toxic reagents are needed in the synthesis process, and the synthesis is simple, environmentally friendly and economical.

It is noted that in Formula (1) of the present application, * denotes the bonding site of the nitrogen atom to the transition metal element M, and in Formula (2), * denotes the bonding site of the Z atom to the transition metal element M. The counterion in the present application serves to balance the charge of the complex formed after coordination of the transition metal element and the ligands, ensuring that the final transition metal complex solution is electrically neutral. The transition metal complex as a whole complies with the law of conservation of positive and negative charge.

It is to be understood that the molecular formula of the transition metal complex in the present application is selected from [M(A)(B)(C)]^{a}bY, [M(A)(D)(E)(F)]^{a}bY, or M(A)(G)(H)(O)(P)^{a}bY. Here, B, C, H, O, and P are merely English letters used as identifiers and do not correspond to chemical elements in the periodic table (e.g., B does not denote boron, C does not denote carbon, H does not denote hydrogen, O does not denote oxygen, and P does not denote phosphorus). In Formula (2), Z₁ and Z₂ are each independently selected from N (nitrogen), P (phosphorus), or As (arsenic).

In one embodiment, B, C, and D are each independently selected from the bidentate ligands having the structure of Formula (1).

Optionally, A is a bidentate ligand having the structure of Formula (3), 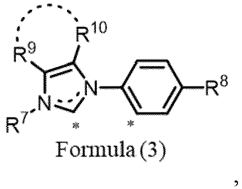 where R⁷ is an alkyl group, a substituted or an unsubstituted benzyl group. In one embodiment, R⁷ is preferably an alkyl group with 1-4 carbon atoms or a benzyl group substituted with at least one electron group, where the electron group is selected from an alkyl group with 1 to 4 carbon atoms, an alkoxy group with 1 to 3 carbon atoms, cyano group, carboxyl group, aldehyde group, or halogen atom. The halogen atom is selected from fluorine, chlorine, bromine, or iodine.

Further, R⁸ is selected from hydrogen, an alkyl group, or an alkoxy group. In one embodiment, R⁸ is preferably an alkyl group with 1 to 2 carbon atoms or an alkoxy group with 1 to 2 carbon atoms. In one embodiment, a covalent bond may or may not be present between R⁹ and R¹⁰. In one embodiment, R⁹ and R¹⁰ are each independently selected from hydrogen, an alkyl group, or an aryl group; in one embodiment, R⁹ and R¹⁰ are preferably alkyl groups with 1 to 2 carbon atoms.

The aforementioned ligand can form a better C-C coordination bond with the transition metal M. The ligand can form two carbon-metal bonds. The ligand is capable of providing stronger electron-donating capacity. The stronger electron-donating ability further reduces the REDOX potential of the transition metal complex. The ligand can enhance the electron transfer capacity of the transition metal complex.

It should be noted that * in equation (3) of the present invention represents the connection site where the carbon atom bonded to the transition metal element M is located.

Optionally, in Formula (1), L₁ and L₂ are each independently selected from a heterocyclic ring containing 1 to 3 nitrogen atoms, and R¹ and R² are each independently selected from an alkyl group with 1 to 3 carbon atoms, an alkoxy group with 1 to 3 carbon atoms, or an alkylamino group with 1 to 6 carbon atoms.

Optionally, in Formula (2), R³, R⁴, R⁵, and R⁶ are each independently selected from an alkyl group with 3 to 6 carbon atoms or an aryl group substituted with at least one electron-donating group with 1 to 4 carbon atoms, where the electron-donating group is an alkyl, alkoxy, or alkylamino group.

Optionally, in the monodentate ligand containing a heterocyclic ring with at least one heteroatom, the heteroatom is oxygen, sulfur, or nitrogen. In one embodiment, the monodentate ligand is selected from at least one of pyridine, imidazole, pyrazole, oxazole, thiazole, pyrazine, triazole, pyrimidine.

Optionally, the transition metal element is selected from Group VIII metals, preferably Fe, Ru, Os, Co, Rh, or Ir, and more preferably Os.

Optionally, the counterion is selected from PF6⁻, BF4 , Cl⁻, I⁻, F⁻, or Br , with PF₆⁻being preferred.

Optionally, the transition metal complex is any compound having a structure selected from Formulas (4) to (10);

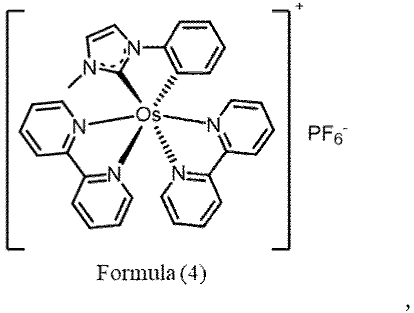

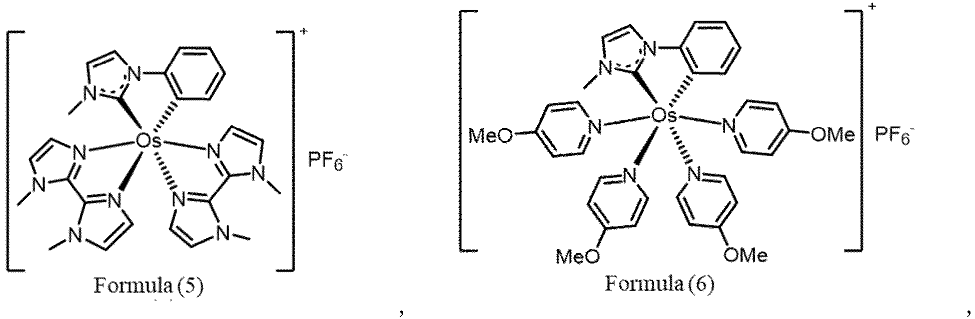

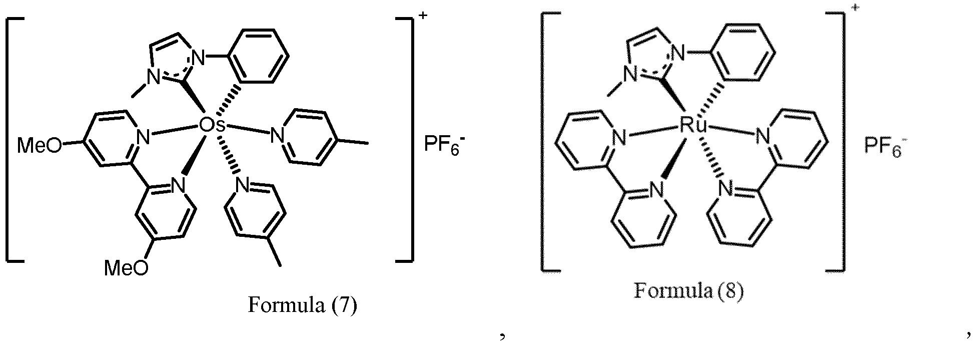

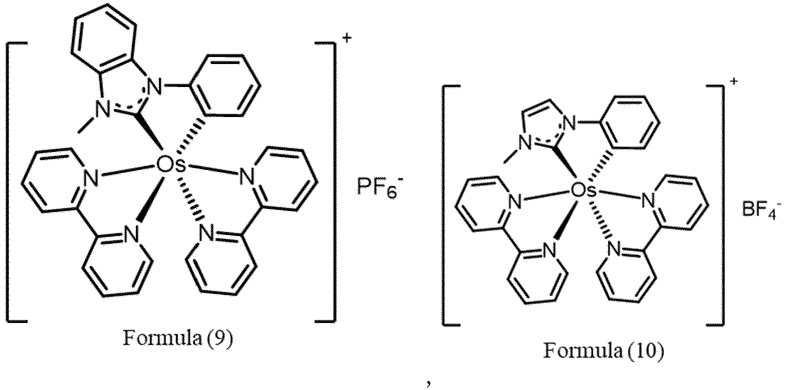

It is noted that the alkyl group as used herein is defined as either a straight-chain or branched-chain alkyl group, with straight-chain alkyl groups being preferred.

At the same time, the present application also provides a method for preparing the transition metal complex as described above, comprising:
S1, Under a protective atmosphere, mix the precursor material of ligand A, an additive, a transition metal dimer, a basic compound, and a first organic solvent. After mixing, react the above materials. After the reaction is complete, perform a separation operation. A transition metal complex precursor containing ligand A is obtained by separation.
S2. Under a protective atmosphere, mix the transition metal complex precursor containing ligand A, a precursor material of ligand S, and a second organic solvent. After mixing, react the above materials. During the reaction, add the counterion precursor material to continue the reaction. After the reaction is complete, sequentially separate and dry the materials. A transition metal complex is obtained through separation and drying. Herein, the ligand S comprises a ligand B and a ligand C, or the ligand S comprises a ligand D, a ligand E, and a ligand F, or the ligand S comprises a ligand G, a ligand H, a ligand O, and a ligand P.

In step S1, the molar ratio of the precursor material of ligand A to the additive is 2:1 to 4:1, the molar ratio of the precursor material of ligand A to the transition metal dimer is 2:1 to 4:1, and the molar ratio of the precursor material of ligand A to the basic compound is 1:2 to 1:80. By setting these ratios, a transition metal complex precursor with C - C ligands can be obtained more efficiently.

Optionally, the additive is selected from at least one of silver oxide, silver trifluoromethanesulfonate, silver carbonate, or molecular sieve.

Optionally, the transition metal dimer is selected from at least one of aryl osmium dimer, dichloroaryl ruthenium dimer. The transition metal dimer is preferably an aryl osmium dimer. The aryl osmium dimer is selected from at least one of dichloryl aryl osmium dimer, phenyl osmium dimer, 1 - methyl - 4 - isopropylphenyl osmium dimer.

Optionally, the basic compound is selected from at least one of basic alumina, sodium carbonate, or sodium bicarbonate.

In one embodiment, the first organic solvent is selected from at least one of dichloromethane, chloroform, 1,2 - dichloroethane.

Optionally, the reaction temperature is 0 °C to 100 °C, preferably 25 °C to 40 °C, and the reaction time is 0.5 h to 100 h, preferably 12 h to 36 h.

In one embodiment, the protective atmosphere is selected from an inert atmosphere and/or a nitrogen atmosphere. The protective atmosphere is preferably a nitrogen atmosphere.

In one embodiment, the steps of the separation operation specifically include: removal of the excess first organic solvent, recrystallization, and filtration. The removal of the excess first organic solvent is carried out by reducing the pressure. The recrystallization is carried out using a dichloromethane / n - hexane system.

In one embodiment, the specific steps of mixing and reacting the precursor material of ligand A, the additive, the transition metal dimer, the basic compound, and the first organic solvent are as follows: The precursor material of ligand A, the additive, and the first organic solvent are mixed and reacted to form a reaction intermediate. The transition metal dimer is then added to form a first carbon-metal bond. Subsequently, the basic compound is added to form a second carbon-metal bond. The excess first organic solvent is then removed under reduced pressure, followed by recrystallization from a dichloromethane/n-hexane system and filtration to obtain the transition metal complex precursor containing ligand A. This process yields a high-purity transition metal complex precursor containing ligand A with two carbon-metal bonds.

In step S2, the molar ratio of the transition metal complex precursor containing ligand A to the precursor material of ligand S is from 1: 1 to 1:4.

Optionally, the molar ratio of the transition metal complex precursor containing ligand A to the counterion precursor material is from 1:10 to 1:30.

Optionally, the second organic solvent is selected from at least one of ethylene glycol, methanol, ethanol.

Optionally, the reaction temperature is from 80°C to 170°C, preferably from 25°C to 40°C, and the reaction time is from 12 h to 72 h, preferably from 12 h to 36 h.

In one embodiment, the protective atmosphere in step S2 is selected from an inert atmosphere and/or a nitrogen atmosphere, preferably a nitrogen atmosphere.

In one embodiment, step S2 further comprises, prior to adding the counterion precursor material, mixing the transition metal complex precursor containing ligand A, the precursor material of ligand S, and the second organic solvent, reacting the mixture to form an intermediate, and treating the intermediate with an anion exchange resin followed by filtration.

The method for preparing the transition metal complex provided by the present application does not require the use of mercury-containing toxic reagents and features simplicity, environmental friendliness, and economic efficiency.

Furthermore, the present application provides an electrochemical biosensor device comprising a sensing membrane that includes a bioanalytical enzyme and an electron transfer medium, wherein the electron transfer medium is the transition metal complex as described herein. The sensing membrane of the electrochemical biosensor device incorporates the transition metal complex of the present application as the electron transfer medium, thereby exhibiting characteristics of high sensitivity and rapid detection speed.

Optionally, the bioanalytical enzyme is selected from oxidase or dehydrogenase, with glucose oxidase being preferred.

In one embodiment, the electrochemical biosensor device further includes electrodes, with the sensing membrane disposed on the surface of the electrodes. The electrodes comprise a working electrode, a counter electrode, and a reference electrode.

The transition metal complex, its preparation method, and its use are further described below through specific embodiments. Those skilled in the art will appreciate that the following embodiments are provided for illustrative purposes only and should not be construed as limiting the scope of the present application. Unless specific conditions are noted, general conditions or those recommended by the manufacturer are employed. Reagents or instruments used, where manufacturer details are not specified, are conventional products available through commercial sources.

It is noted that Examples 1 to 7 of the present application and the structural formulas of the complex corresponding to Proportion 1 are set forth in Formulas (4)-(10) and (15), respectively:

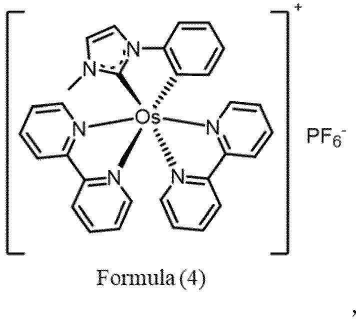

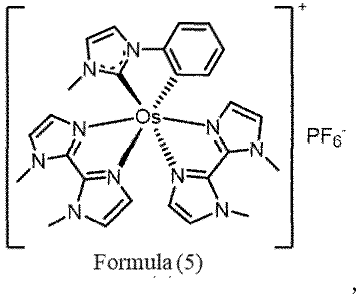

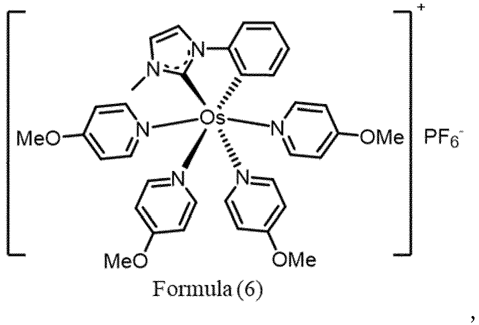

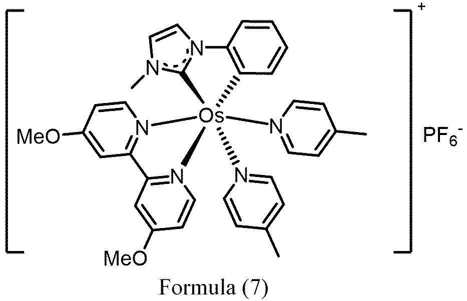

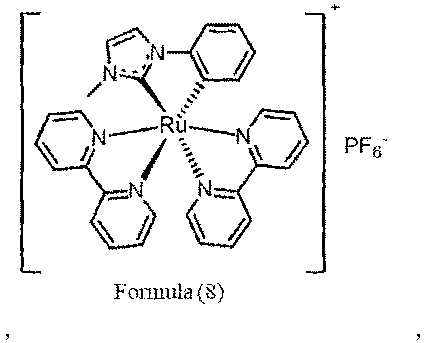

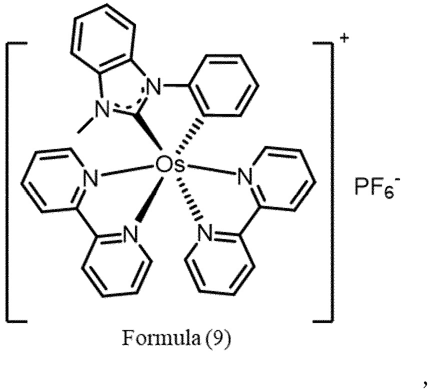

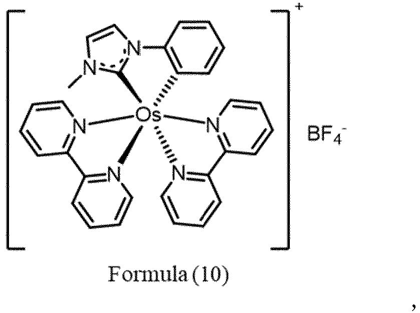

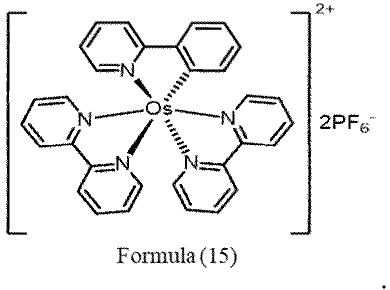

### Example 1

Under a nitrogen atmosphere, 1 - methyl - 3 - phenylimidazole iodized salt (287.1 mg, 1 mmol), silver oxide (115.9 mg, 0.5 mmol), and activated 4Å molecular sieve (250 mg) were added to a dry 100 - mL three - necked flask. The three - necked flask was wrapped with tin foil, then dichloromethane (20 mL) was added, and the mixture was placed in the dark at room temperature and reacted for 1 hour. Then, dichloroaryl osmium dimer (395.4 mg, 0.5 mmol) was added and stirring was continued for 3 hours. Basic alumina (4 g) was added and the reaction was continued with stirring for 24 hours. The excess dichloromethane solvent was removed under reduced pressure. Recrystallization was carried out using a dichloromethane / n - hexane system, and filtration yielded 341.2 mg of osmium complex precursor, with a yield of 64%.

Under a nitrogen atmosphere, the obtained osmium complex precursor (103.6 mg, 0.2 mmol), 2,2' - bipyridine (62.5 mg, 0.4 mmol), and ethylene glycol (4 mL) were added to a dry 50 - mL necked flask and mixed. The mixture was then refluxed at 140 °C for 24 hours and cooled to room temperature after the reaction was completed. AG1X4Cl - resin (2.5 g) and deionized water (12 mL) were added, and the mixture was stirred open at room temperature for 24 hours. After the reaction was completed, the mixture was filtered and the filtrate was dropped into a rapidly stirred ammonium hexafluorophosphate aqueous solution (0.7 g, 10 mL of deionized water), and the solid was filtered. The solid was redissolved in acetonitrile (1.5 mL), and the above operation was repeated. The filtered solid was placed in a vacuum drying oven at 50 °C for 24 hours to obtain 90.3 mg of osmium complex, with a yield of 56%. The structural formula of the osmium complex is shown in Formula (4).

The reaction formula for the osmium complex prepared by Example 1 is as follows:

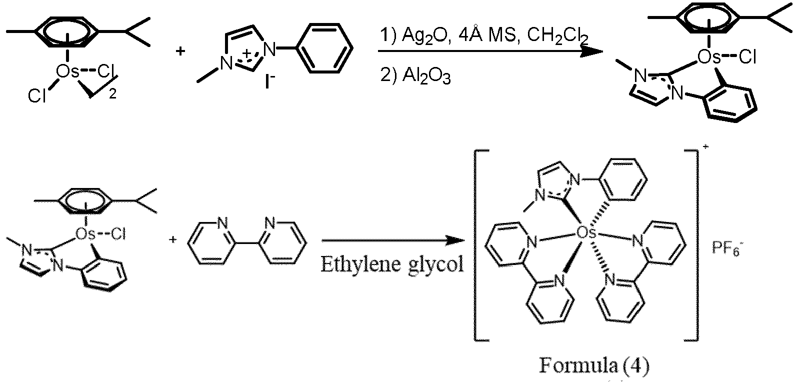

The osmium complex precursor prepared in Example 1 was tested by nuclear magnetic resonance spectroscopy (NMR) using hydrogen NMR (¹H - NMR (400 MHz, DMSO-d₆)) and carbon - 13 NMR (¹³C - NMR (100 MHz, DMSO-d₆)). The NMR data from the ¹H - NMR test are as follows: δ7.75 to 7.44 (m, 4H), δ6.42 (s, 1H), δ6.00 (s, 1H), δ5.73 to 5.54 (m, 2H), δ5.48 to 5.25 (m, 2H), δ4.03 (s, 3H), δ2.25 (s, 3H), δ2.15 to 2.06 (m, 1H), δ1.04 (d, J = 7.2 Hz, 3H), δ0.98 (d, J = 7.2 Hz, 3H); in ¹³C - NMR, δ173.2, δ140.6, δ136.2, δ127.6, δ124.3, δ122.9, δ122.5, δ121.6, δ120.4, δ100.2, δ83.5, δ76.1, δ74.3, δ34.2, δ30.9, δ23.1, δ18.9. From the NMR data, it can be seen that δ7.75 to 7.44 ppm belongs to the hydrogen of pyridine, δ6.42 and δ6.00 belong to the hydrogen of the carbene ring, and δ1.04 and δ0.98 belong to the hydrogen of isopropyl. It can be seen that the osmium complex precursor prepared in Example 1 of the present application contains C - C ligands, pyridine, and 1 - methyl - 4 - isopropylphenyl groups, indicating that the osmium complex precursor has been successfully synthesized.

Meanwhile, the osmium complex prepared in Example 1 was analyzed by an organic elemental analyzer, and the calculated mass percentages of C, H, and N elements in C₃₀H₂₆F₆N₆OsP in the entire molecular formula are: C, 44.72; H, 3.25; N, 10.43; the experimental values are: C, 44.8; H, 3.68; N, 10.59. It can be seen from this that the osmium complex in Example 1 of the present application has been successfully synthesized.

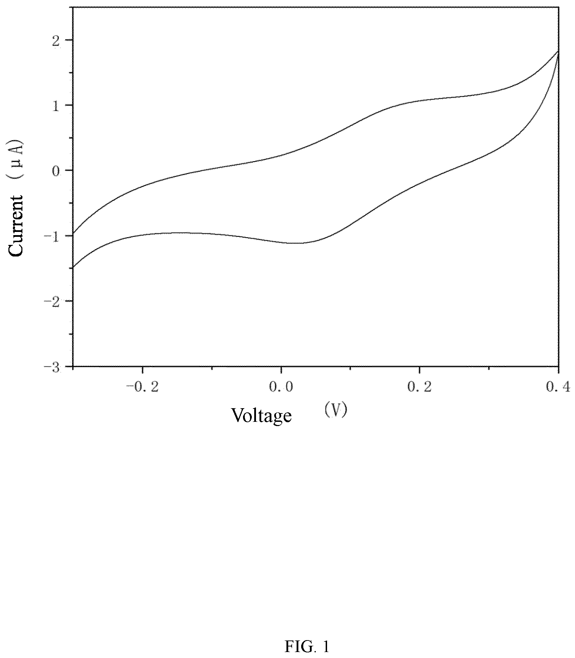

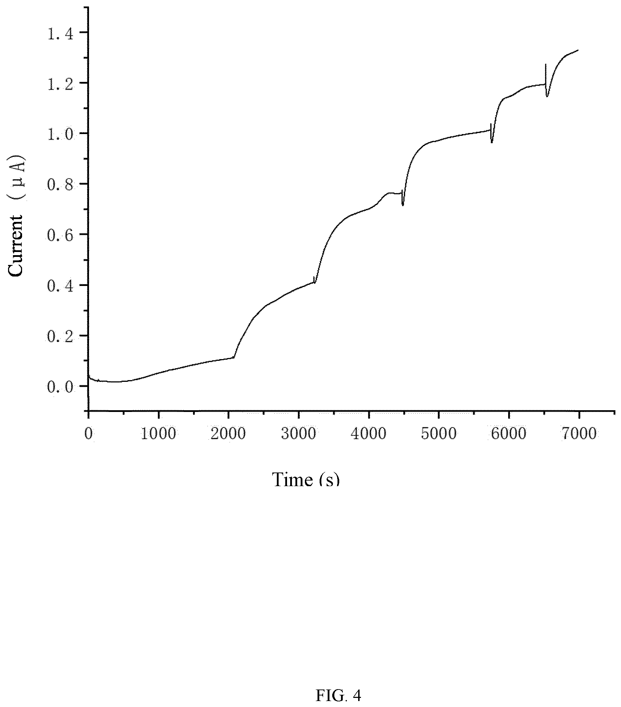

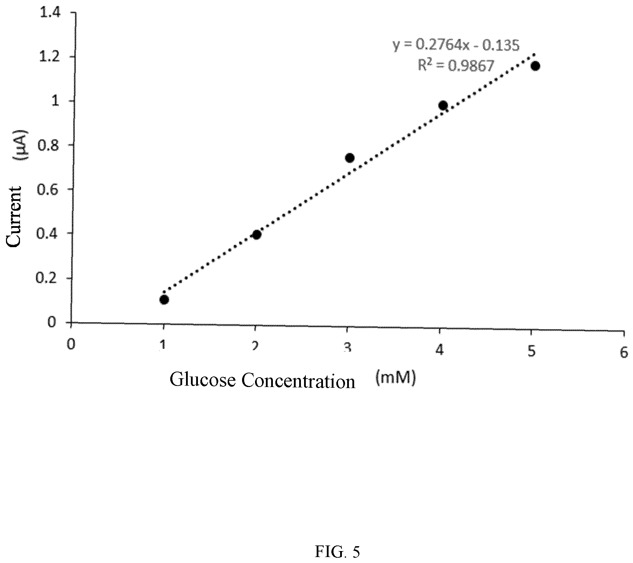

In addition, as can be seen from FIG. 1, the osmium complex prepared in Example 1 has a lower redox potential and is suitable for use in a glucose sensor. As can be seen from FIGS. 4 to 5, when the osmium complex prepared in Example 1 is used as an electron transfer medium, it can participate in the cyclic reaction between the enzyme and glucose, form a catalytic current, and has good electrocatalytic performance and high sensitivity for glucose.

### Example 2

The sole difference from Example 1 is as follows: Under otherwise identical conditions, 1,1'-dimethyl-1H,1'H-[2,2']biimidazole (64.9 mg, 0.4 mmol) is used in place of 2,2'-bipyridine (62.5 mg, 0.4 mmol), yielding 101.3 mg of the osmium complex with a 62% yield. The structural formula of the osmium complex is shown in Formula (5) of the present application.

The reaction formula for preparing the osmium complex in this embodiment is as follows:

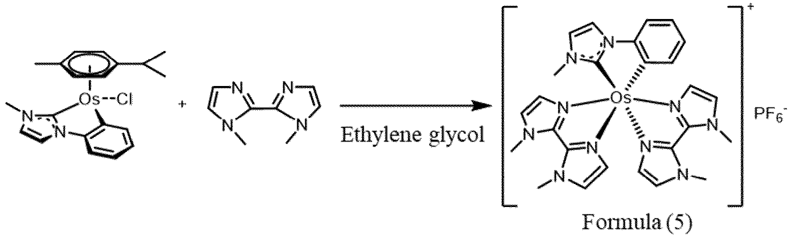

The osmium complex prepared in Example 2 was analyzed using an organic elemental analyzer. For the molecular formula C₂₆H₃₀F₆N₁₀OsP, the calculated mass percentages of C, H, and N are: C, 38.19; H, 3.70; N, 17.13. The experimental values are: C, 38.10; H, 3.88; N, 17.35. This confirms the successful synthesis of the osmium complex of Example 2.

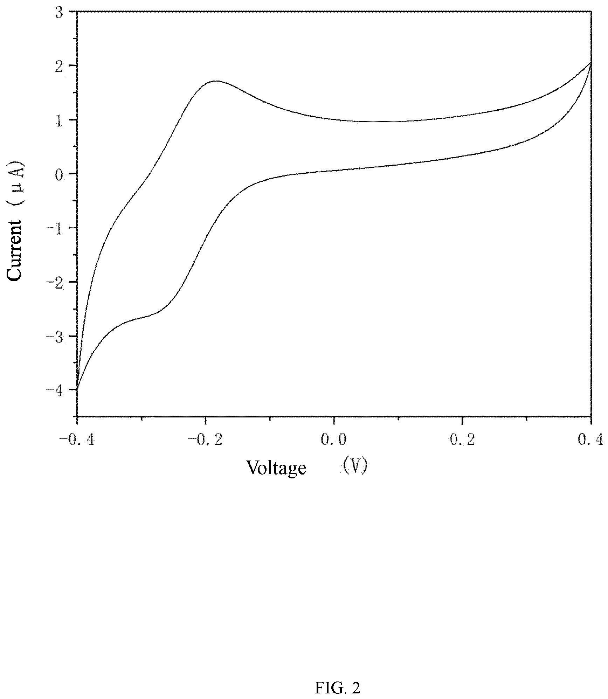

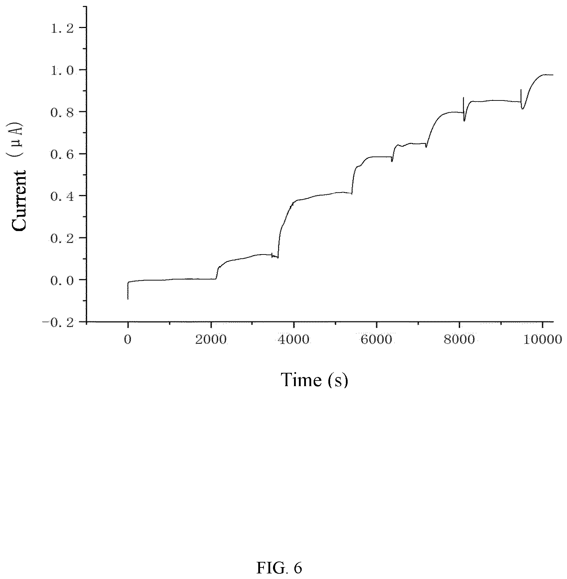

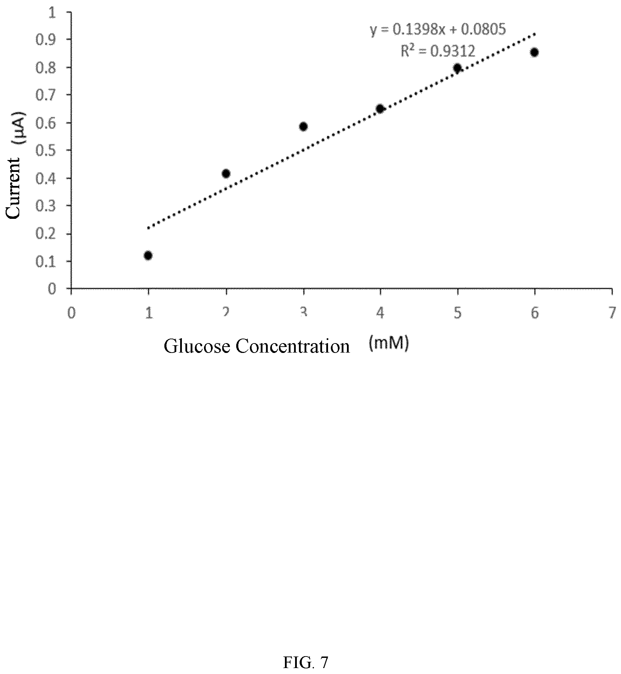

As shown in FIG. 2, the osmium complex prepared in Example 2 exhibits a relatively low redox potential, making it suitable for use in glucose sensors. As depicted in FIGS. 6 to 7, when employed as an electron transfer medium, the osmium complex of Example 2 participates in the cyclic reaction between the enzyme and glucose, generates a catalytic current, and demonstrates excellent electrocatalytic performance, high sensitivity, and strong anti-interference capability toward glucose.

### Example 3

The only distinction from Example 1 is the substitution of 2,2'-bipyridine (62.5 mg, 0.4 mmol) with 4-methoxypyridine (87.3 mg, 0.8 mmol), with all other conditions remaining unchanged. This yields 103.9 mg of the osmium complex with a 56% yield, and its structural formula is shown in Formula (6) of the present application.

### Example 4

The sole variation from Example 1 is the use of 4,4'-dimethoxy-2,2'-bipyridine (43.2 mg, 0.2 mmol) and 4-methylpyridine (37.3 mg, 0.4 mmol) in place of 2,2'-bipyridine (62.5 mg, 0.4 mmol), with identical remaining conditions. This results in 115.6 mg of the osmium complex with a 74% yield, and its structural formula is shown in Formula (7) of the present application.

### Example 5

Example 5 differs from Example 1 only in that a dichloroaryl ruthenium dimer (312.2 mg, 0.5 mmol) is used instead of the dichloroaryl osmium dimer (395.4 mg, 0.5 mmol), with all other conditions unchanged. This produces 210.3 mg of the ruthenium complex precursor with a 47% yield. Using the method of Example 1, the ruthenium complex precursor (90.0 mg, 0.2 mmol), 1,1'-dimethyl-1H, 1'H-[2,2']bisimidazole (64.9 mg, 0.4 mmol), and ethylene glycol (4 mL) are added to a dry 50 mL three-necked flask for mixing and reaction under identical conditions, yielding 77.4 mg of the ruthenium complex with a 52% yield. The structural formula of the ruthenium complex is shown in Formula (8).

### Example 6

The only difference between Example 6 and Example 1 is the use of 1-methyl-3-phenylbenzimidazole iodide (337.2 mg, 1 mmol) instead of 1-methyl-3-phenylimidazole iodide (287.1 mg, 1 mmol), with all other conditions unchanged. This yields 396.6 mg of the osmium complex precursor with a 68% yield. The osmium complex is then prepared using the method of Example 1, resulting in 73.6 mg of the osmium complex with a 43% yield, and its structural formula is shown in Formula (9).

### Example 7

Example 7 differs from Example 1 solely in the use of ammonium tetrafluoroborate instead of the ammonium hexafluorophosphate aqueous solution (0.7 g, 10 mL deionized water)), with all other conditions identical. This yields 80.0 mg of the osmium complex with a 44% yield, and its structural formula is shown in Formula (10).

### Example 8 (Proportional Example 1)

The sole difference between Proportional Example 1 and Example 1 is as follows: 4-phenylpyridine (155.2 mg, 1 mmol) is used in place of 1-methyl-3-phenylimidazole iodide (287.1 mg, 1 mmol), and dichloroaryl osmium dimer (395.4 mg, 0.5 mmol), basic alumina (4 g) and dichloromethane (20 mL) are directly added. After continuing to stir and react with for 24 hours, 353.9 mg of the osmium complex precursor is obtained with a 66% yield. The osmium complex is then prepared using the method of Example 1, yielding 79.5 mg of the osmium complex with a 42% yield, and its structural formula is shown in Formula (15).

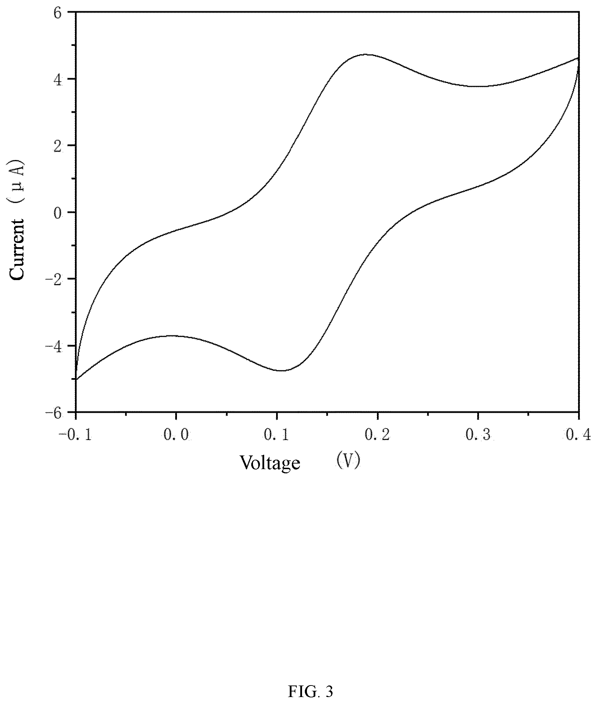

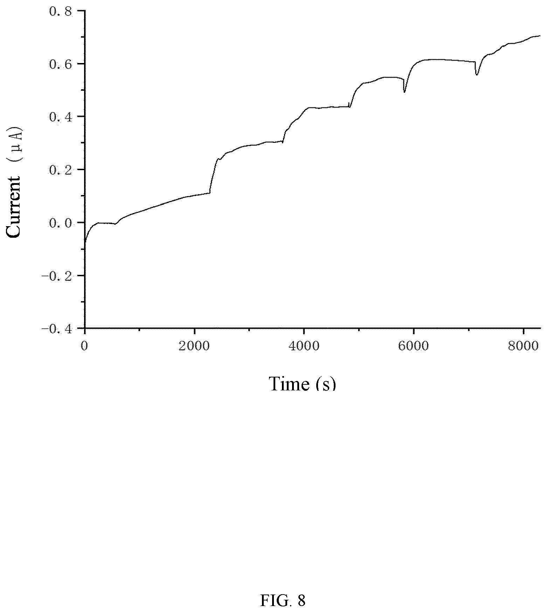

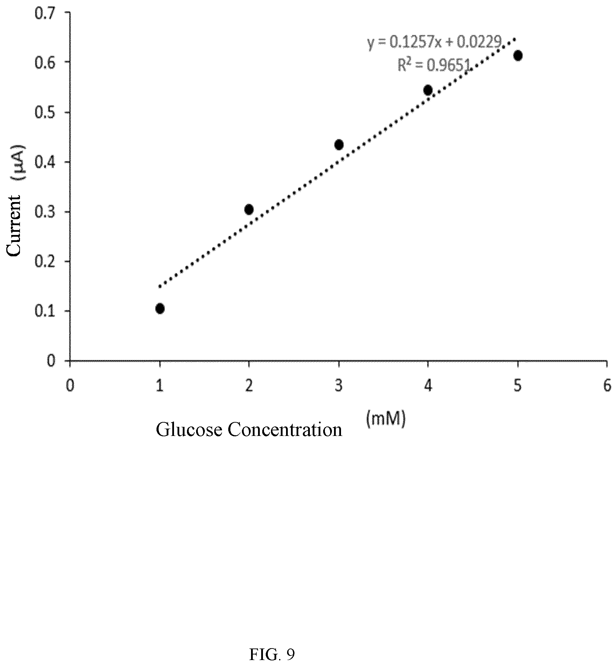

As evident from FIGS. 1 to 3, the redox potential of the osmium complex prepared in Proportional Example 1 is higher than that of Examples 1 to 2. As shown in FIGS. 4 to 9, while the osmium complex of Proportional Example 1 can also function as an electron transfer medium, its electron transfer efficiency is lower due to the presence of C-N ligands and absence of C-C ligands, resulting in poorer sensitivity.

### Cyclic Voltammetry and Reaction Rate Constant Measurements

Cyclic voltammetry was performed to measure the redox potentials of Examples 1 to 4, 6 to 7, the osmium complex from Proportional Example 1, and the ruthenium complex from Example 5. The procedure was as follows: The complex under test was dissolved in acetonitrile to prepare a 30 mg/mL solution, which was coated on a glassy carbon working electrode. Using Ag/AgCl as the reference electrode and platinum as the counter electrode, measurements were conducted in a pH 7.4 buffer solution at a sweep rate of 50 mV/s. The results are presented in Table 1.

Additionally, the osmium complexes from Examples 1 to 4, 6 to 7, and the osmium complex from Proportional Example 1, the ruthenium complex from Example 5 were incorporated into sensing membranes for electrochemical biosensor devices, and their reaction rate constants with glucose oxidase were measured. The specific steps were: Each complex and glucose were dissolved in a pH 7.4 buffer to form a mixed solution (complex concentration: 10⁻⁴ M, glucose concentration: 50 mM). Cyclic voltammetry was performed at different scan rates using Ag/AgCl as the reference electrode, a platinum counter electrode, and a glassy carbon working electrode. Glucose oxidase was then added to a final concentration of 10⁻⁶ M, and measurements were repeated at different scan rates. The results are shown in Table 1.

**Table 1**

| | Exam ple 1 | Exam ple 2 | Exam ple 3 | Exam ple 4 | Exam ple 5 | Exam ple 6 | Exam ple 7 | Example 8 (Propor tional Example 1) |
|---|---|---|---|---|---|---|---|---|
| *E*_{1/2} *vsAg*/*AgCl* (mV) | 90 | -230 | 24 | -71 | 45 | 160 | 120 | 174 |
| Reaction Rate Constant (*M*^{*-*1}*s*⁻¹) | 0.3 × 10⁶ | 19.2 × 10⁶ | 17.1 × 10⁶ | 6.2 × 10⁶ | 4.6 × 10⁶ | 10.3 × 10⁶ | 5.5 × 10⁶ | 4.1 × 10⁶ |

As demonstrated in Table 1 and FIGS. 1 to 9, the transition metal complex of the present application exhibits a low, tunable redox potential, avoiding interference from most substances when used in electrochemical biosensors. It demonstrates excellent electron transfer ability and rate, good electrocatalytic performance toward reactants, and high sensitivity.

The various technical features disclosed in the foregoing embodiments may be combined in any manner. For brevity, not all possible combinations of these technical features are explicitly described herein. Nevertheless, any combination of the technical features that does not create a contradiction is intended to be within the scope of this specification.

The embodiments described above represent only a subset of the present application and are presented with particularity for clarity. They should not be interpreted as limiting the broad scope of the application. It is to be understood that those skilled in the art may make various alterations and modifications without departing from the spirit and scope of the application, all of which are intended to fall within the protection of the present application. Accordingly, the scope of the present application is defined solely by the appended claims.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present application, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claim subject matter lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate a certain variation (e.g., ±1%, ±5%, ±10%, or ±20%) of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable. In some embodiments, a classification condition used in classification or determination is provided for illustration purposes and modified according to different situations. For example, a classification condition that "a value is greater than the threshold value" may further include or exclude a condition that "the probability value is equal to the threshold value".

## Claims

1. A transition metal complex, wherein the transition metal complex formula is selected from:
[M(A)(B)(C)]^{a}bY,[M(A)(D)(E)(F)]^{a}bY, or [M(A)(G)(H)(O)(P)]^{a}bY; wherein:
M is a transition metal element;
A is a bidentate ligand containing a benzene ring and a carbene heterocyclic ring with at least one nitrogen atom;
B, C, and D are each independently a bidentate ligand selected from the group consisting of a structure represented by Formula (1) and a structure represented by Formula (2), as shown below;
E, F, G, H, O, and P are each independently a monodentate ligand selected from a heterocyclic ring containing at least one heteroatom, CN⁻, or Cl⁻;
a represents the number of positive charges and a is 0 or 1; Y is a counterion; b represents the number of counterions and b is 0 or 1; 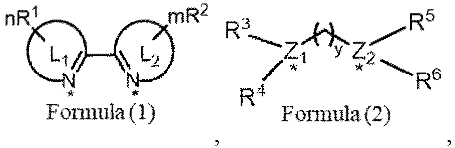
in Formula (1), L₁ and L₂ are each independently selected from heterocycles containing at least one nitrogen atom,
R¹ and R² are each independently selected from a hydrogen atom, an alkyl group, an alkoxy group, or an alkylamino group, and
n and m are each independently selected from integers from 0 to 5;
in Formula (2), Z₁ and Z₂ are each independently selected from N, P, or As;
R³, R⁴, R⁵, and R⁶are each independently selected from an alkyl group, a substituted or an unsubstituted aryl group, and
y is an integer from 1 to 4.

2. The transition metal complex in claim 1, wherein:
A is a bidentate ligand having the structure shown in Formula (3), 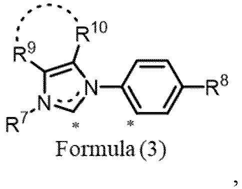
in Formula (3), R⁷ is selected from an alkyl group, a substituted benzyl group or an unsubstituted benzyl group;
R⁸is selected from a hydrogen atom, an alkyl group, or an alkoxy group, and
there may or may not be a covalent bond between R⁹ and R¹⁰, and
R⁹ and R¹⁰ are each independently selected from a hydrogen atom, an alkyl group, or an aryl group.

3. The transition metal complex in claim 2, wherein:
R⁷ is selected from an alkyl group with 1 to 4 carbon atoms or a benzyl group with at least one hydrogen atom replaced by an electron group, wherein:
the electron group is selected from an alkyl group with 1 to 4 carbon atoms, an alkoxy group with 1 to 3 carbon atoms, a cyano group, a carboxyl group, an aldehyde group, or a halogen atom.

4. The transition metal complex in claim 2, wherein:
R⁸ is selected from an alkyl group with 1 to 2 carbon atoms or an alkoxy group with 1 to 2 carbon atoms.

5. The transition metal complex in claim 2, wherein:
R⁹ and R¹⁰ are each independently selected from alkyl groups with 1 to 2 carbon atoms.

6. The transition metal complex as claimed in claim 1, wherein:
R¹ and R² are each independently selected from an alkyl group with 1 to 3 carbon atoms, an alkoxy group with 1 - 3 carbon atoms, and an alkylamino group with 1 to 6 carbon atoms.

7. The transition metal complex in claim 1, wherein:
R³, R⁴, R⁵, and R⁶ are each independently selected from an alkyl group with 3 to 6 carbon atoms or an aryl group with at least one hydrogen atom replaced by an electron-donating group with 1 to 4 carbon atoms, and the electron-donating group is selected from an alkyl group, an alkoxy group, or an alkylamino group.

8. The transition metal complex in claim 1,wherein:
the heterocyclic ring containing at least one heteroatom is selected from at least one of pyridine, imidazole, pyrazole, oxazole, thiazole, pyrazine, triazole, pyrimidine.

9. The transition metal complex in claim 1, wherein:
the transition metal element is selected from Fe, Ru, Os, Co, Rh, or Ir.

10. The transition metal complex in claim 1, wherein:
the counterion is selected from at least one of PF₆⁻, BF₄⁻, Cl⁻, I⁻, F⁻, or Br⁻.

11. The transition metal complex in claim 1, wherein the transition metal complex is any of the compounds having the structure shown in Formulas (4) to (10):

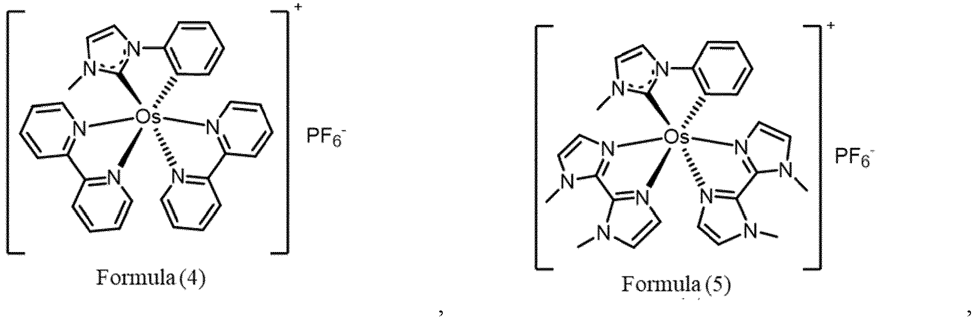

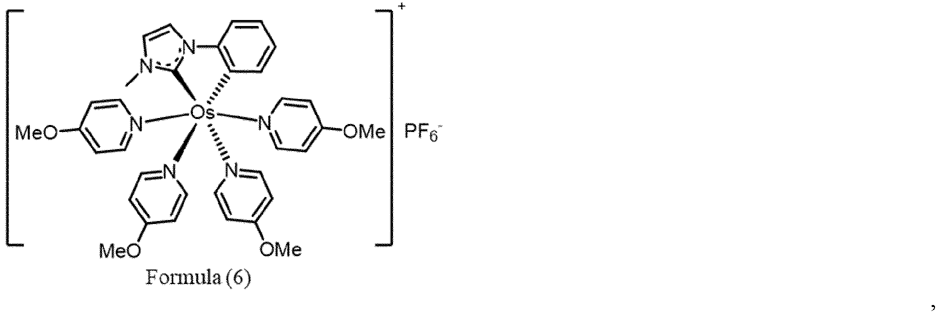

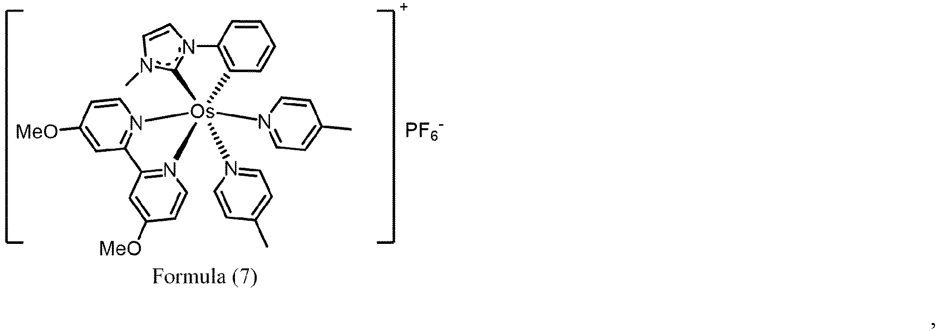

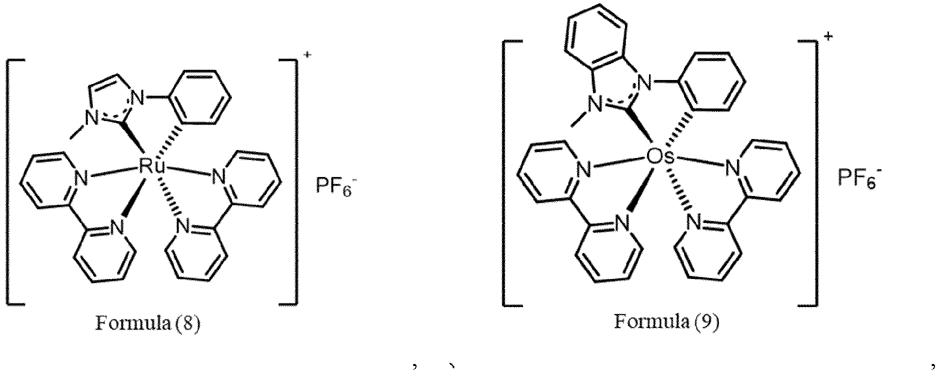

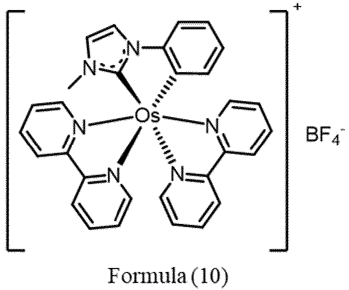

12. A method for preparing the transition metal complex in claim 1, wherein, the method comprises:
under a protective atmosphere, mixing and reacting the precursor material of ligand A, an additive, a transition metal dimer, a basic compound, and a first organic solvent, and separating to obtain the precursor of the transition metal complex containing ligand A;
under a protective atmosphere, mixing and reacting the precursor of the transition metal complex containing ligand A, a precursor material of ligand S, and a second organic solvent, then adding the counterion precursor material for reaction, and after separation and drying, obtaining the transition metal complex;
wherein, the ligand S comprises a ligand B and a ligand C, or the ligand S comprises a ligand D, a ligand E, and a ligand F, or the ligand S comprises a ligand G, a ligand H, a ligand O, and a ligand P.

13. The method for preparing the transition metal complex in claim 12, wherein at least one of the following conditions must be met in the steps for preparing the transition metal complex:
(1) the molar ratio of the precursor material of ligand A to the additive is 2:1 - 4:1, the molar ratio of the precursor material of ligand A to the transition metal dimer is 2:1 to 4:1, and the molar ratio of the precursor material of ligand A to the basic compound is 1:2 to 1:80;
(2) the additive is selected from at least one of silver oxide, silver trifluoromethanesulfonate, silver carbonate, or molecular sieve, the transition metal dimer is selected from aryl osmium dimer, and the basic compound is selected from at least one of basic aluminum oxide, sodium carbonate, or sodium bicarbonate;
(3) in the steps of mixing and reacting the precursor material of ligand A, the additive, the transition metal dimer, the basic compound, and the first organic solvent, the reaction temperature is 25 °C to 40 °C and the reaction time is 12h to 36h;
(4) the molar ratio of the transition metal complex precursor containing ligand A to the precursor material of ligand S is 1: 1 to 1:4;
(5) the molar ratio of the transition metal complex precursor containing ligand A to the counterion precursor material is 1: 10 to 1:30;
(6) in the steps of mixing and reacting the transition metal complex precursor containing ligand A, the precursor material of ligand S, and the second organic solvent, the second organic solvent is selected from at least one of ethylene glycol, methanol, and ethanol, the reaction temperature is 25 °C to 40 °C, and the reaction time is 12 h to 36 h.

14. An electrochemical biosensor device, wherein the electrochemical biosensor device comprises a sensing membrane, the sensing membrane comprising a bioanalytical enzyme and an electron transfer medium, wherein the electron transfer medium is the transition metal complex in claim 1.

15. The electrochemical biosensor device in claim 14, wherein the bioanalytical enzyme is selected from an oxidase or a dehydrogenase.
